(19) Europäisches Patentamt
European Patent Office
Office européen des brevets

(11) EP 4 474 382 A1

(12) EUROPEAN PATENT APPLICATION
published in accordance with Art. 153(4) EPC

(43) Date of publication:
**11.12.2024 Bulletin 2024/50**

(21) Application number: **23747173.5**

(22) Date of filing: **31.01.2023**

(51) International Patent Classification (IPC):
***C07D 413/10*** *(2006.01)* ***C09K 9/02*** *(2006.01)*
***G02C 7/10*** *(2006.01)* ***C07D 311/04*** *(2006.01)*
***C09B 57/00*** *(2006.01)*

(52) Cooperative Patent Classification (CPC):
**C07D 311/04; C07D 413/10; C09B 57/00; C09K 9/02; G02C 7/10**

(86) International application number:
**PCT/JP2023/003028**

(87) International publication number:
**WO 2023/145963 (03.08.2023 Gazette 2023/31)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **31.01.2022 JP 2022013482**

(71) Applicant: **HOYA LENS THAILAND LTD.**
**Pathumthani 12130 (TH)**

(72) Inventors:
- **KAWAKAMI, Hironori**
  **Tokyo 160-8347 (JP)**
- **KOBAYASHI, Kei**
  **Tokyo 160-8347 (JP)**
- **HIGUCHI, Takeshi**
  **Tokyo 160-8347 (JP)**

(74) Representative: **Hoffmann Eitle**
**Patent- und Rechtsanwälte PartmbB**
**Arabellastraße 30**
**81925 München (DE)**

# (54) PHOTOCHROMIC COMPOUND, PHOTOCHROMIC COMPOSITION, PHOTOCHROMIC ARTICLE, AND EYEGLASSES

(57) Provided is a photochromic compound represented by General Formula 1. In General Formula 1, R represents a substituted or unsubstituted ring structure which is spirofused with an indeno-fused naphthopyran and has 3 or more and 20 or less carbon atoms (including the carbon atom at the 13-position of the indeno-fused naphthopyran), and $R^1$ to $R^4$, B, and B' each independently represent a hydrogen atom or a substituent.

EP 4 474 382 A1

General Formula 1

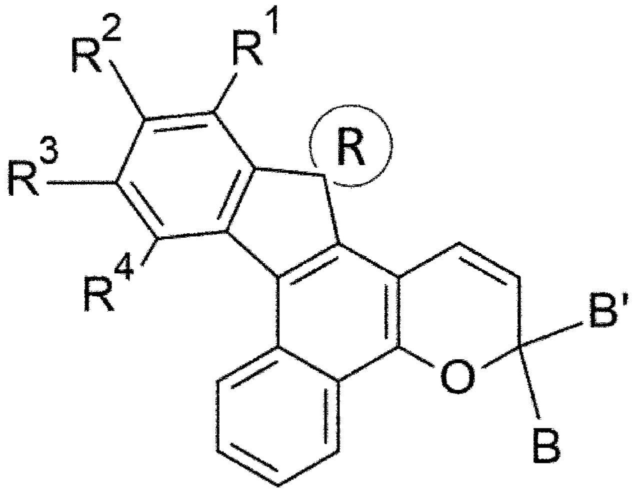

## Description

[Technical Field]

The present invention relates to a photochromic compound, a photochromic composition, a photochromic article, and spectacles.

[Background Art]

Photochromic compounds are compounds that have properties of coloring under emission of light in the photoresponsive wavelength range and fading without light emission (photochromic properties). For example, PTL 1 discloses a naphthopyran compound having photochromic properties.

[Citation List]

[Patent Literature]

[PTL 1] WO 2001/60811 A1

[Summary of Invention]

[Technical Problem]

Examples of methods of imparting photochromic properties to optical articles such as spectacle lenses include a method of incorporating a photochromic compound into a substrate and a method of forming a layer containing a photochromic compound. Examples of properties desired for optical articles with photochromic properties include exhibiting a high fading rate after being colored by light emission.

An object of one aspect of the present invention is to provide a photochromic article with a high fading rate.

[Solution to Problem]

The present inventors have conducted extensive studies, and as a result, have newly found that a photochromic compound represented by General Formula 1 having a structure shown below can exhibit a high fading rate.

In other words, one aspect of the present invention relates to a photochromic compound represented by General Formula 1 below.

In addition, one aspect of the present invention relates to a photochromic article containing one or more photochromic compounds represented by General Formula 1 below.

In addition, one aspect of the present invention relates to a photochromic composition containing one or more photochromic compounds represented by General Formula 1 below.

[Chem. 1]

General Formula 1

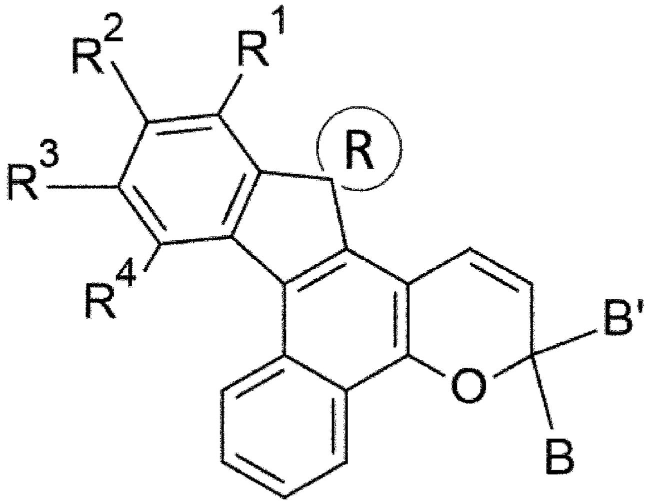

In General Formula 1, R represents a substituted or unsubstituted ring structure which is spirofused with an indeno-fused naphthopyran and has 3 or more and 20 or less carbon atoms (including the carbon atom at the 13-position of the indeno-fused naphthopyran), and $R^1$ to $R^4$, B, and B' each independently represent a hydrogen atom or a substituent.

[Advantageous Effects of Invention]

The photochromic compound represented by General Formula 1 can exhibit a high fading rate. According to the compound represented by General Formula 1, it is possible to provide a photochromic article with a high fading rate.

[Description of Embodiments]

As an example, a photochromic compound undergoes structural conversion into a colored body through an excited state upon emission of light such as sunlight. The structure after structural conversion through light emission can be called a "colored body." On the other hand, the structure before light emission may be called a "colorless body." However, regarding the colorless body, the term "colorless" is not limited to being completely colorless, but also encompasses a case of colors lighter than that of the colored body. The structure of General Formula 1 is a structure of the colorless body.

In the present invention and the present specification, the term "photochromic article" refers to an article containing a photochromic compound. The photochromic article according to one aspect of the present invention contains at least one or more photochromic compounds represented by General Formula 1 as photochromic compounds. The photochromic compounds can be incorporated into a substrate of a photochromic article and/or can be incorporated into a photochromic layer in a photochromic article having a substrate and the photochromic layer. The term "photochromic layer" is a layer containing a photochromic compound.

In the present invention and the present specification, the term "photochromic composition" refers to a composition containing a photochromic compound. The photochromic composition according to one aspect of the present invention contains at least one or more photochromic compounds represented by General Formula 1 as photochromic compounds and can be used for producing a photochromic article according to one aspect of the present invention.

In the present invention and the present specification, substituents in various general formulae to be described in detail below and substituents when each group to be described below has a substituent can each independently be the following substituents:

a substituent $R^m$ selected from the group consisting of 1-18C linear or branched alkyl groups such as a hydroxy group, a methyl group, an ethyl group, a propyl group, a butyl group, a pentyl group, and a hexyl group, 5-18C cycloaliphatic alkyl groups of single-ring types or multi-ring types, such as a bicyclic ring, such as a cyclopentyl group and a cyclohexyl group, linear or branched alkoxy groups having 1 to 24 constituent atoms such as a methoxy group, an

ethoxy group, and a butoxy group, non-aromatic cyclic substituents having 1 to 24 constituent atoms, 1-18C linear or branched perfluoroalkyl groups such as a trifluoromethyl group, linear or branched perfluoroalkoxy groups such as a trifluoromethoxy group, linear or branched alkyl sulfide groups having 1 to 24 constituent atoms such as a methyl sulfide group, an ethyl sulfide group, and a butyl sulfide group, aryl groups such as a phenyl group, a naphthyl group, an anthracenyl group, a fluoranthenyl group, a phenanthryl group, a pyranyl group, a perylenyl group, a styryl group, and a fluorenyl group, aryloxy groups such as a phenyloxy group, aryl sulfide groups such as a phenyl sulfide group, heteroaryl groups such as a pyridyl group, a furanyl group, a thienyl group, a pyrrolyl group, a benzofuranyl group, a benzothiophenyl group, an indolyl group, a dibenzofuranyl group, a dibenzothiophenyl group, a carbazolyl group, a diazolyl group, a triazolyl group, a quinolinyl group, a phenothiazinyl group, a phenoxazinyl group, a phenazinyl group, a thianthryl group, and an acridinyl group, an amino group ($-NH_2$), monoalkylamino groups such as a monomethylamino group, dialkylamino groups such as a dimethylamino group, monoarylamino groups such as a monophenylamino group, diarylamino groups such as a diphenylamino group, cyclic amino groups such as a piperidino group, a morpholino group, a thiomorpholino group, a tetrahydroquinolino group, and a tetrahydroisoquinolino group, an ethynyl group, a mercapto group, a silyl group, a sulfonic acid group, an alkylsulfonyl group, a formyl group, a carboxy group, a cyano group, and halogen atoms such as a fluorine atom, a chlorine atom, a bromine atom, and an iodine atom; or

a substituent in which $R^m$ is further substituted with one or more of the same or different $R^m$'s.

As an example of the above-described substituent in which $R^m$ is further substituted with one or more of the same or different $R^m$'s, a structure can be exemplified in which a terminal carbon atom of an alkoxy group is further substituted with an alkoxy group and a terminal carbon atom of this alkoxy group is further substituted with an alkoxy group. In addition, as another example of the above-described substituent in which $R^m$ is further substituted with one or more of the same or different $R^m$'s, a structure can be exemplified in which two or more positions among five substitutable positions of a phenyl group are substituted with the same or different $R^m$'s. However, the present invention is not limited to such examples.

In the present invention and the present specification, the terms "the number of carbon atoms" and "the number of constituent atoms" refer to the numbers including the number of carbon atoms or the number of atoms of a substituent when referring to a group having a substituent, unless otherwise specified. In addition, in the present invention and the present specification, the expression "unsubstituted or having one or more substituents" has the same meaning as "substituted or unsubstituted."

In addition, in the present invention and the present specification, substituents in various general formulae to be described in detail below and substituents when each group to be described below has a substituent can each independently be solubilizing groups. In the present invention and the present specification, the term "solubilizing group" refers to a substituent that can contribute to increasing the compatibility with any liquid or a specific liquid. As solubilizing groups, substituents, such as 4-50C alkyl groups having a linear, branched or cyclic structure, linear, branched, or cyclic alkoxy groups having 4 to 50 constituent atoms, linear, branched, or cyclic silyl groups having 4 to 50 constituent atoms, those in which some of the above-described groups are substituted with a silicon atom, a sulfur atom, a nitrogen atom, a phosphorus atom, or the like, and a combination of two or more of the above-described groups, that can contribute to promoting thermal motion of molecules of compounds according to inclusion of these substituents are preferable. A compound having a solubilizing group as a substituent can prevent a solute from solidifying by inhibiting the distance between solute molecules from getting closer together, or can create a molecular aggregation state close to that of a liquid by lowering the melting point and/or glass transition temperature of the solute. Thus, the solubilizing group can liquefy a solute or increase the solubility of a compound having this substituent in a liquid. In one form, the solubilizing group is preferably an n-butyl group, an n-pentyl group, an n-hexyl group, and an n-octyl group which are linear alkyl groups, a tert-butyl group which is a branched alkyl group, or a cyclopentyl group and a cyclohexyl group which are cyclic alkyl groups.

The above-described substituent is preferably a substituent selected from the group consisting of a methoxy group, an ethoxy group, a phenoxy group, a methyl sulfide group, an ethyl sulfide group, a phenyl sulfide group, a trifluoromethyl group, a phenyl group, a naphthyl group, a dibenzofuranyl group, a dibenzothiophenyl group, a carbazolyl group, a phenothiazinyl group, a phenoxazinyl group, a phenazinyl group, an acridinyl group, a dimethylamino group, a diphenylamino group, a piperidino group, a morpholino group, a thiomorpholino group, a cyano group, and a solubilizing group, and more preferably a substituent selected from the group consisting of a methoxy group, a phenoxy group, a methyl sulfide group, a phenyl sulfide group, a trifluoromethyl group, a phenyl group, a dimethylamino group, a diphenylamino group, a piperidino group, a morpholino group, a thiomorpholino group, a cyano group, and a solubilizing group.

In the present invention and the present specification, the term "electron-withdrawing group" refers to a substituent that attracts electrons easily from the side of atoms bonded compared to hydrogen atoms. Electron-withdrawing groups can attract electrons as a result of substituent effects such as an inductive effect, a mesomeric effect (or a resonance effect). Specific examples of electron-withdrawing groups include halogen atoms (fluorine atom: -F, chlorine atom: -Cl, bromine atom: -Br, iodine atom: -I), trifluoromethyl group: $-CF_3$, nitro group: $-NO_2$, cyano group: -CN, formyl

group: -CHO, acyl group: -COR (R is a substituent), alkoxycarbonyl group: - COOR, carboxy group: -COOH, substituted sulfonyl group: $-SO_2R$ (R is a substituent), and sulfo group: $-SO_3H$. Examples of preferred electron-withdrawing groups include a fluorine atom which is an electron-withdrawing group having high electronegativity and an electron-withdrawing group having a positive value for the substituent constant $\sigma_p$ at the para-position based on Hammett's rule.

In the present invention and the present specification, the term "electron-donating group" refers to a substituent that more easily donates electrons to the side of atoms bonded compared to hydrogen atoms. The electron-donating group can be a substituent that easily donates electrons due to the sum of an inductive effect, a mesomeric effect (or a resonance effect), and the like. Specific examples of electron-donating groups include a hydroxy group: -OH, a thiol group: -SH, an alkoxy group: -OR (R is an alkyl group), an alkyl sulfide group: -SR (R is an alkyl group), an aryl sulfide group, an acetyl group: $-OCOCH_3$, an amino group: $-NH_2$, an alkylamide group: $-NHCOCH_3$, a dialkylamino group: $-N(R)_2$ (where two R's are the same or different alkyl groups), and a methyl group. Examples of suitable electron-donating groups include an electron-donating group having a negative value for the substituent constant $\sigma_p$ at the para-position based on Hammett's rule.

Specific examples of the substituent constant $\sigma_p$ at the para-position based on Hammett's rule (source: Daigakuin Yuki Kagaku (Jou) ("Graduate School Organic Chemistry (Part 1)"

(1988) edited by Hiizu Iwamura, Ryoji Noyori, Takeshi Nakai, and Isao Kitagawa) are shown below.

$-N(CH_3)_2$: -0.83
$-OCH_3$: -0.27
$-t-C_4H_9$: -0.20
$-CH_3$: -0.17
$-C_2H_5$: -0.15
$-C_6H_5$: -0.01
(-H: 0)
-F: +0.06
-Cl: +0.27
-Br: +0.23
$-CO_2C_2H_5$: +0.45
$-CF_3$: +0.54
-CN: +0.66
$-SO_2CH_3$: +0.72
$-NO_2$: +0.78

[Photochromic compound represented by General Formula 1]

Hereinafter, a photochromic compound represented by General Formula 1 will be described in more detail.

[Chem. 2]

General Formula 1

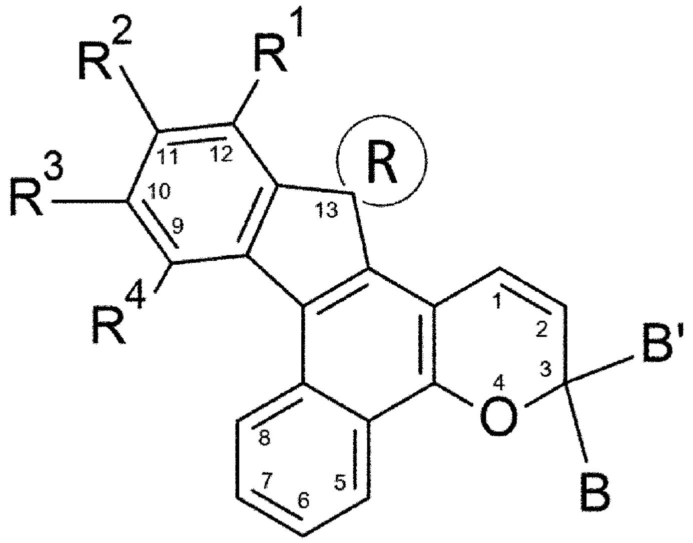

In General Formula 1, R represents a substituted or unsubstituted ring structure which is spirofused with an indeno-fused naphthopyran and has 3 or more and 20 or less carbon atoms (including the carbon atom at the 13-position of the indeno-fused naphthopyran). The carbon atom at the 13-position of indenonaphthopyran is a spiro atom shared by the indeno-fused naphthopyran and the ring structure represented by R is a ring structure spirofused with the indeno-fused naphthopyran.

The ring structure represented by R can be unsubstituted or can have a substituent. When R represents a ring structure having a substituent, the above-described number of carbon atoms refers to the number of carbon atoms including the number of carbon atoms of the substituent. The number of carbon atoms of the ring structure represented by R (including the carbon atom at the 13-position of the indeno-fused naphthopyran) is 3 or more, and can be 4 or more, 5 or more, 6 or more, or 7 or more. In addition, the number of carbon atoms of the ring structure represented by R (including the carbon atom at the 13-position of the indeno-fused naphthopyran) is 20 or less, and can be 19 or less, 18 or less, 17 or less, 16 or less, or 15 or less.

The ring structure represented by R can be a substituted or unsubstituted alicyclic structure. Such an alicyclic structure can be a monocyclic structure, can be a condensed polycyclic structure such as a bicyclic or tricyclic structure, can be a bridged ring structure such as a bicyclic structure, and can be a spirocyclic structure such as a bicyclic structure.

Specific examples of the following partial structure in General Formula 1 include the following partial structures and partial structures included in exemplary compounds listed below.

[Chem. 3]

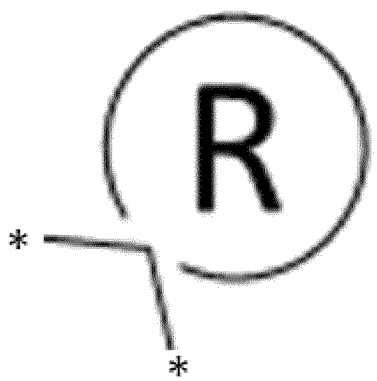

In the present invention and the present specification, the symbol "*" for a partial structure of a compound indicates a bonding position with an atom to which such a partial structure is bonded.

[Chem. 4]

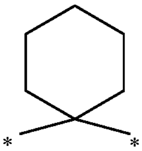
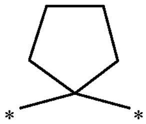
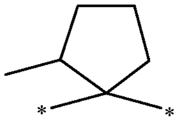
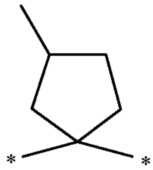
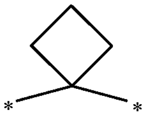
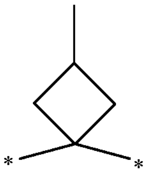
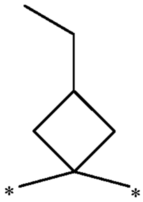
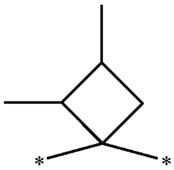
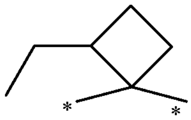
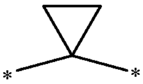
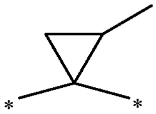
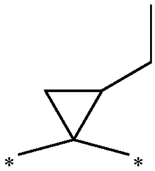
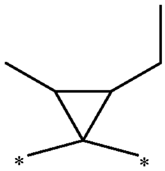
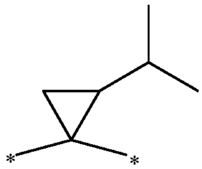
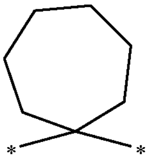
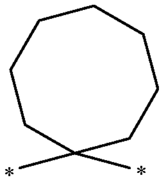
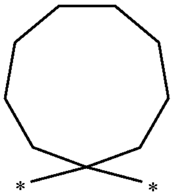
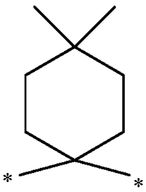
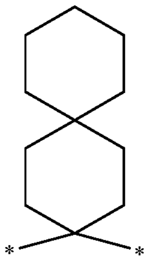
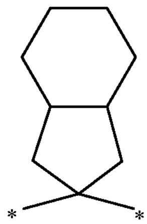
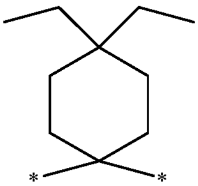
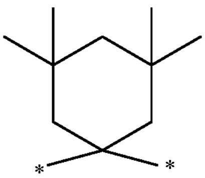

General Formula 1, $R^1$ to $R^4$, B, and B' each independently represent a hydrogen atom or a substituent. Substituents are as described previously.

In one form, $R^1$ to $R^4$ can all represent a hydrogen atom.

In General Formula 1, $R^1$ to $R^4$ can represent a hydrogen atom or a substituent other than an electron-withdrawing group. Examples of substituents other than an electron-withdrawing group include an electron-donating group, and the details thereof are as described previously.

B and B' in General Formula 1 each independently represent a hydrogen atom or a substituent, and it is preferable that they each independently represent a substituted or unsubstituted phenyl group, a substituted or unsubstituted naphthyl group, a substituted or unsubstituted fluorenyl group, a substituted or unsubstituted benzofluorenyl group, a substituted or unsubstituted fluoranthenyl group, a substituted or unsubstituted dibenzofuranyl group, or a substituted or unsubstituted dibenzothiophenyl group. It is more preferable that B and B' each independently represent a substituted or unsubstituted phenyl group. Such a substituted phenyl group can have one or more substituents selected from the group consisting of a methoxy group, an isopropyl group, a tert-butyl group, a methyl sulfide group, an amino group, a dimethylamino group, a piperidino group, a morpholino group, a thiomorpholino group, a phenyl group, fluorine atom, a chlorine atom, a bromine atom, an iodine atom, a trifluoromethyl group, and a cyano group.

Examples of photochromic compounds represented by General Formula 1 include the following compounds. However, the present invention is not limited to the compounds exemplified below.

[Chem. 5]

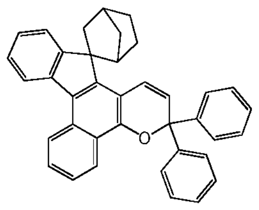
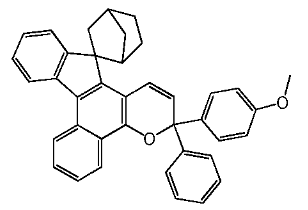
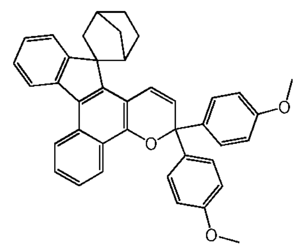

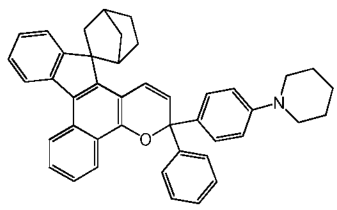
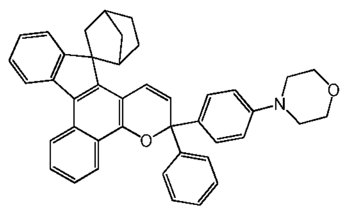
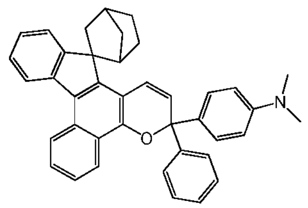

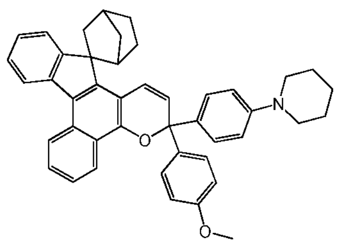
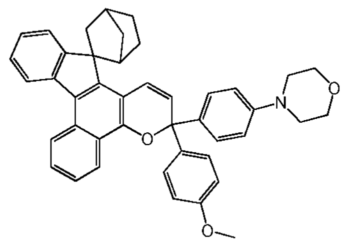
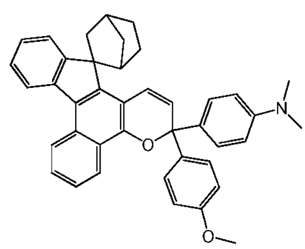

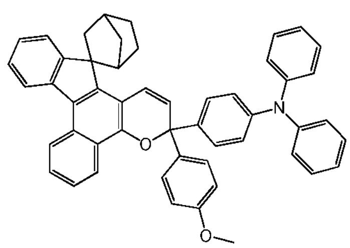
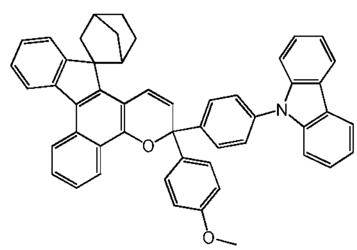
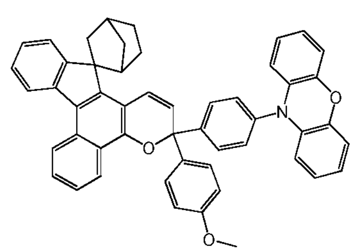

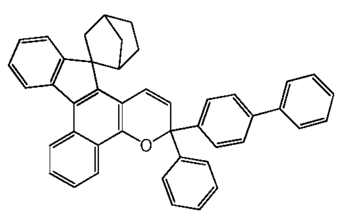
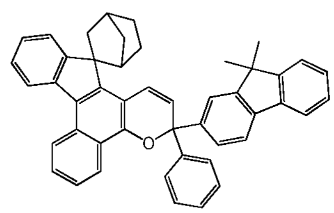
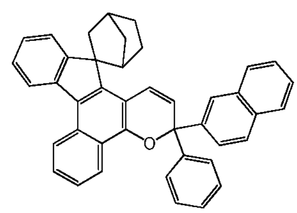

[Chem. 6]

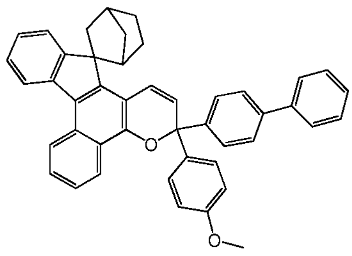
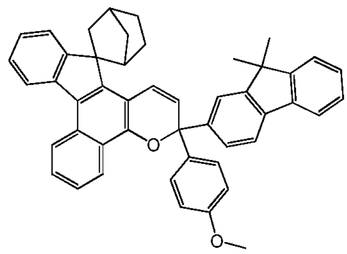
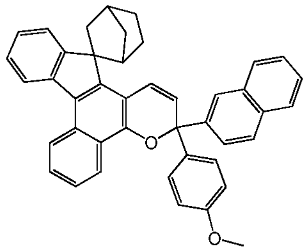
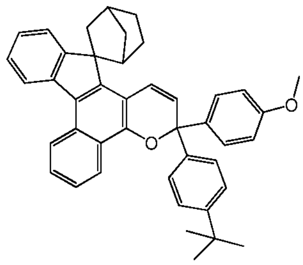
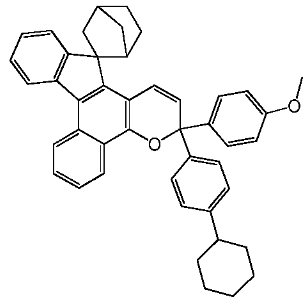
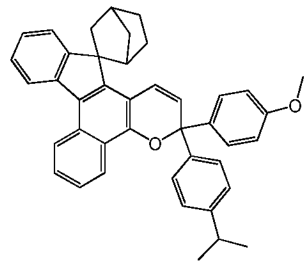
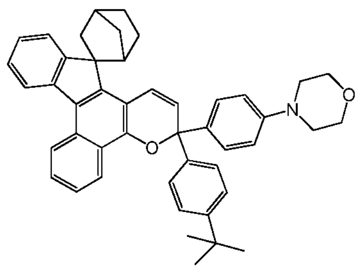
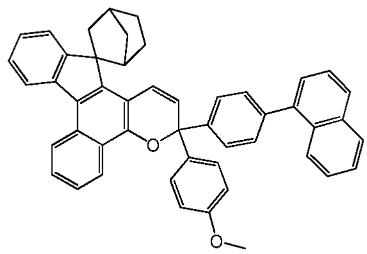
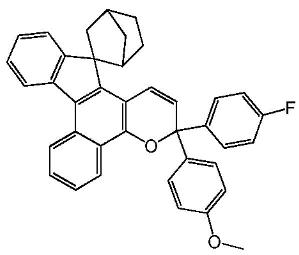
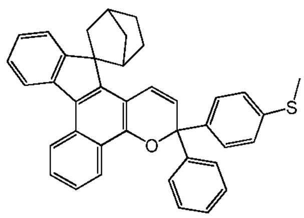
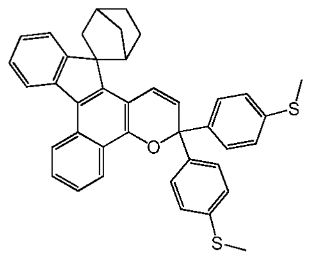
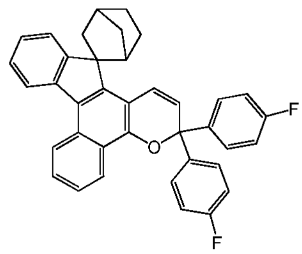
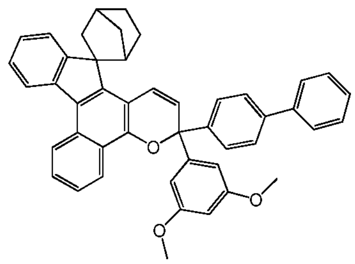
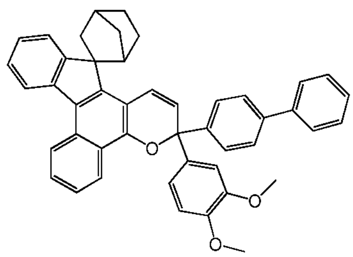

[Chem. 7]

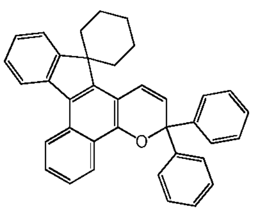

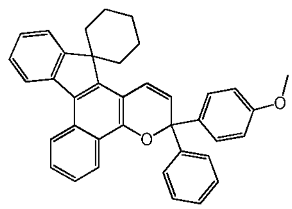

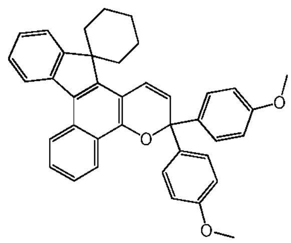

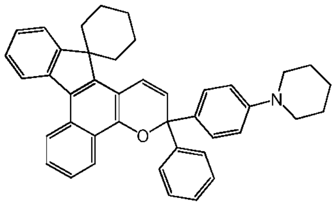

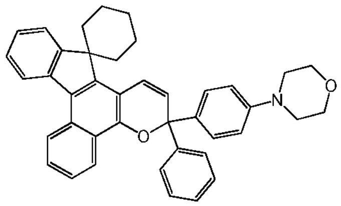

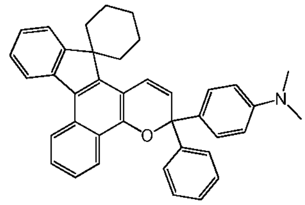

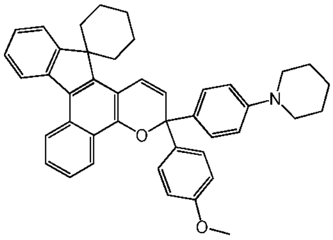

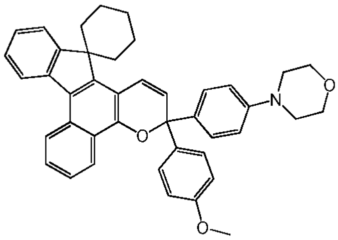

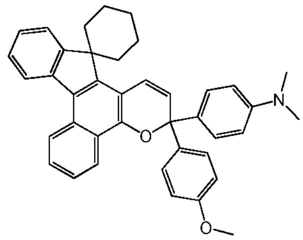

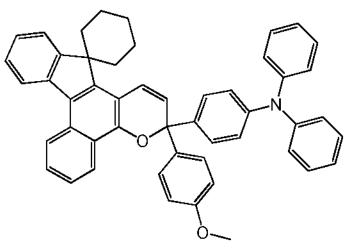

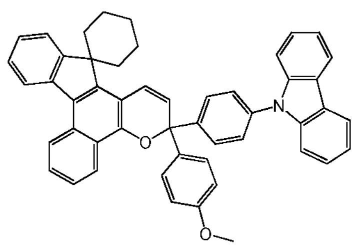

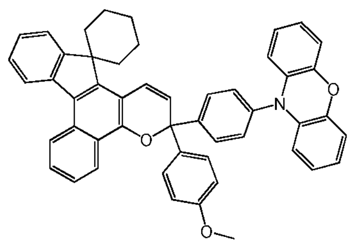

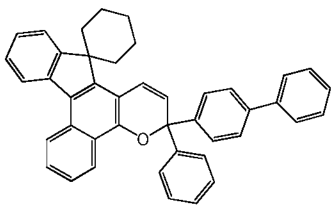

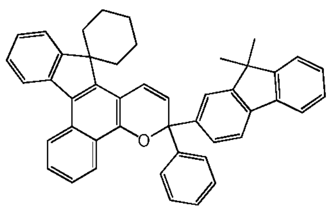

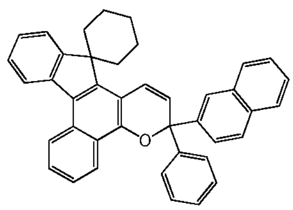

[Chem. 8]

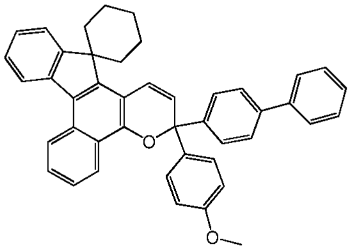
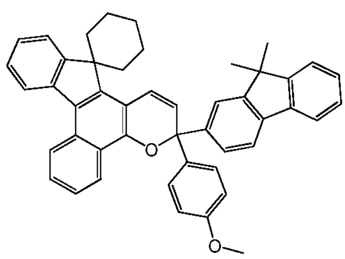
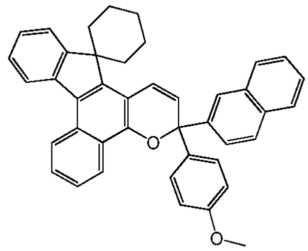
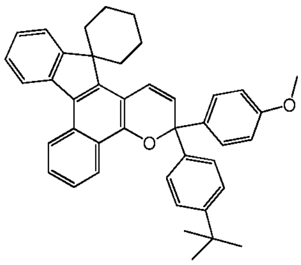
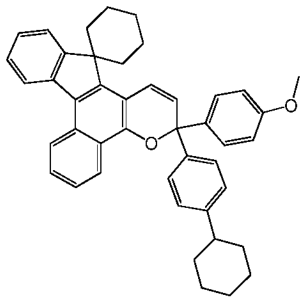
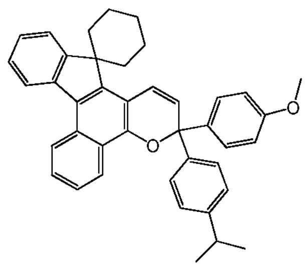
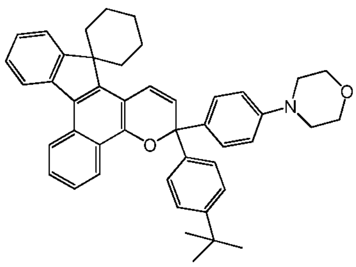
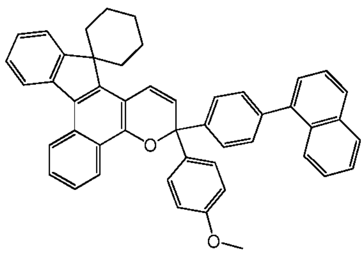
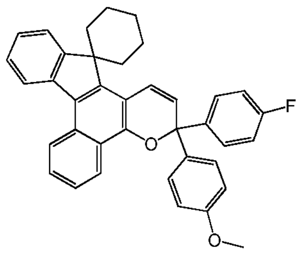
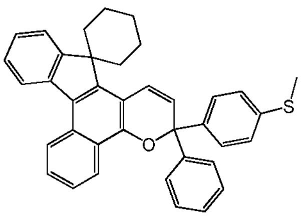
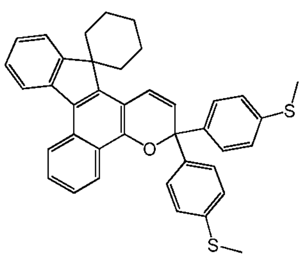
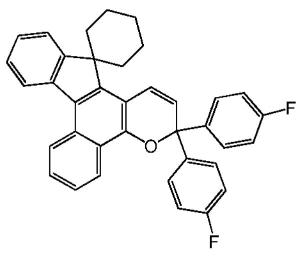
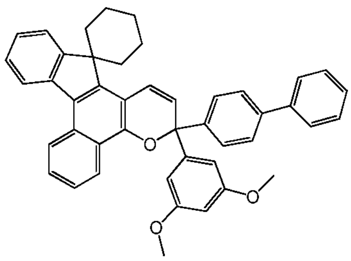
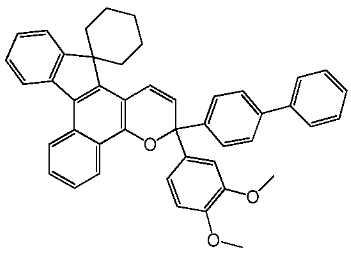

[Chem. 9]

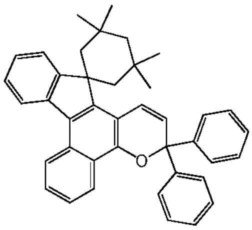
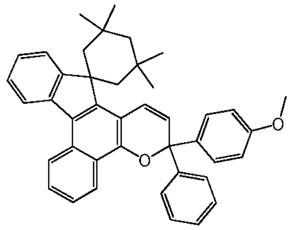
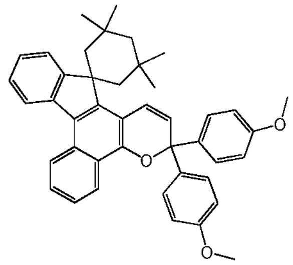
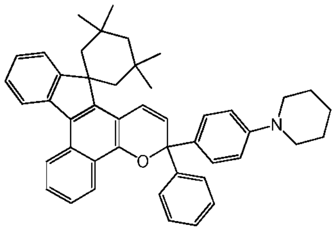
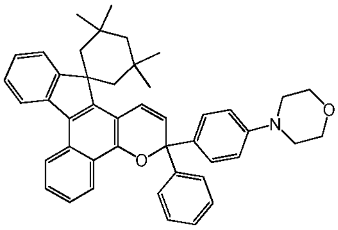
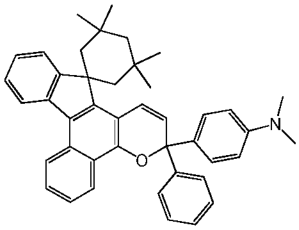
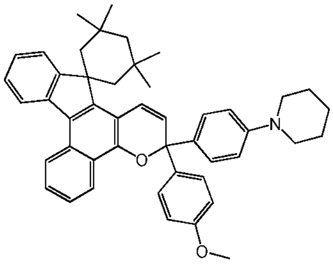
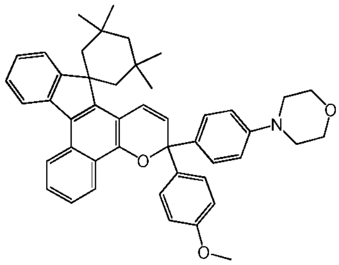
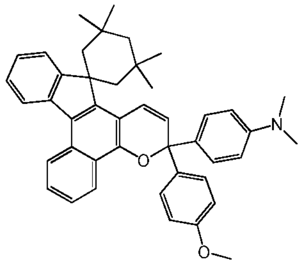
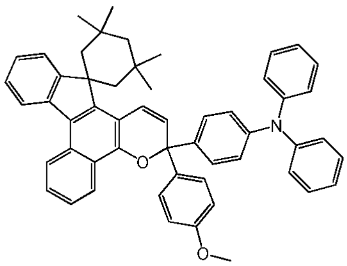
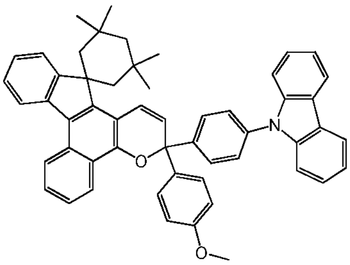
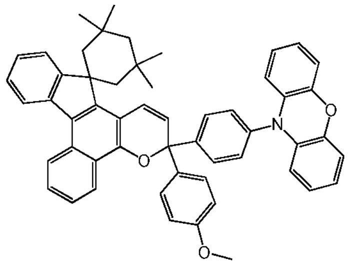
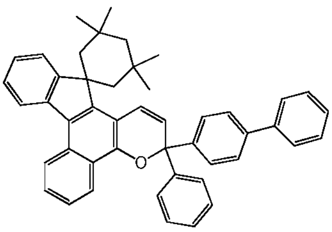
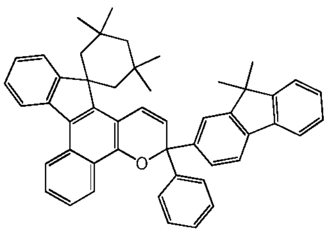
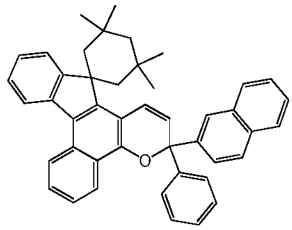

[Chem. 10]

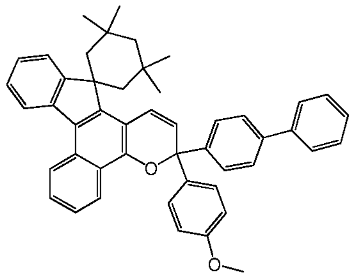

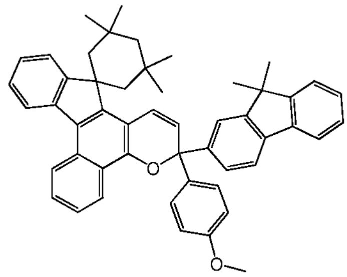

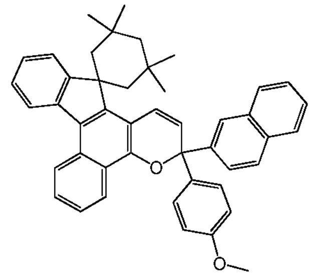

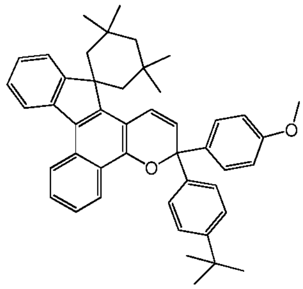

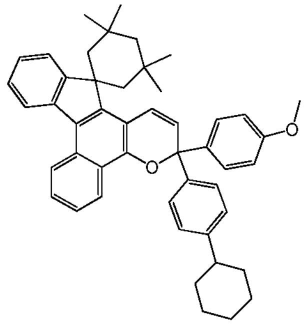

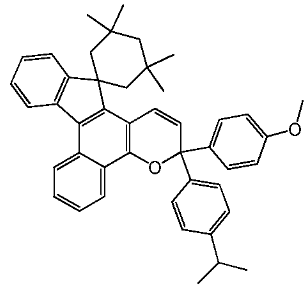

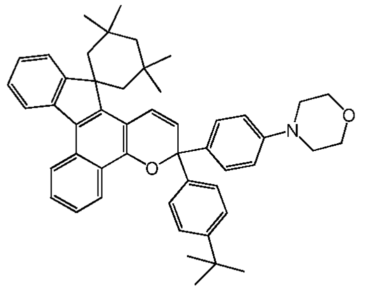

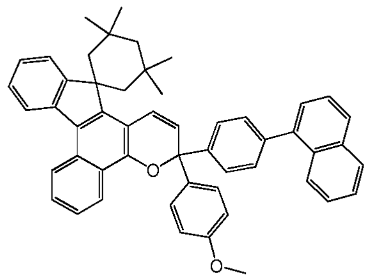

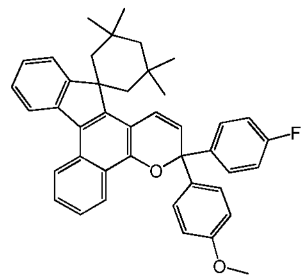

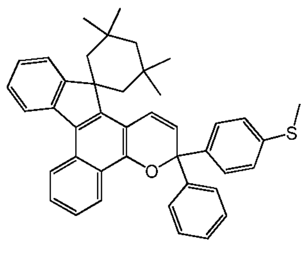

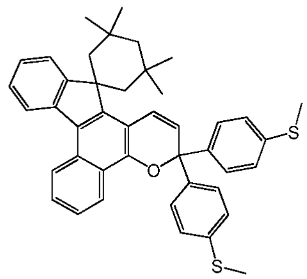

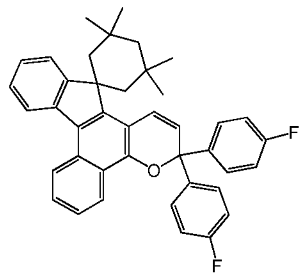

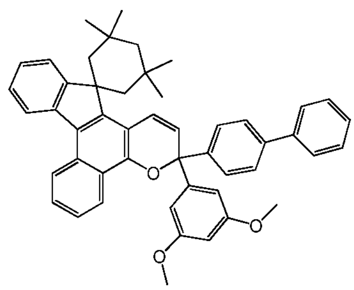

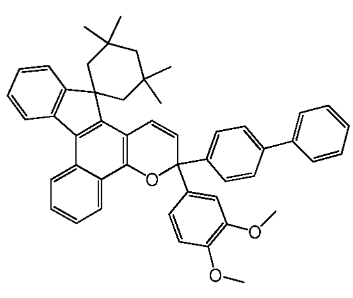

[Chem. 11]

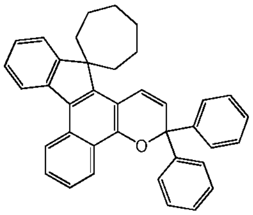
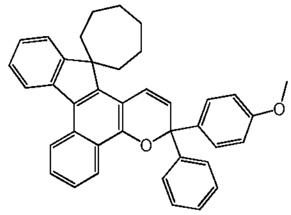
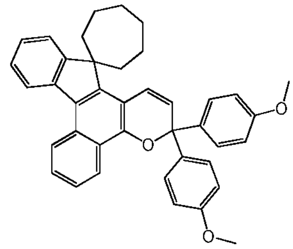
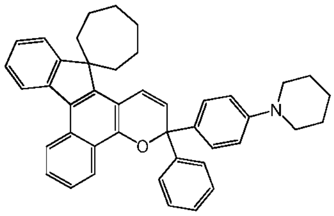
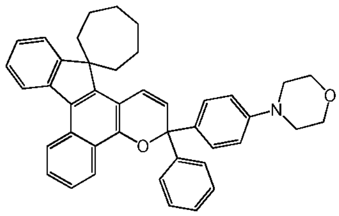
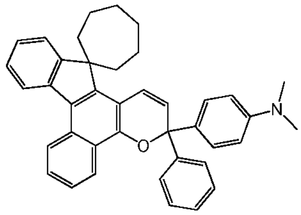
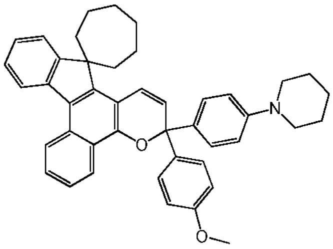
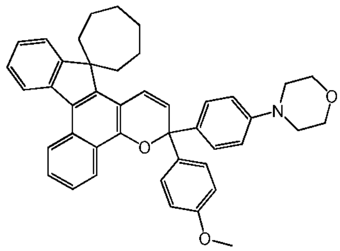
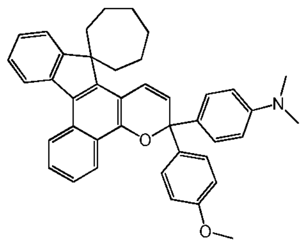
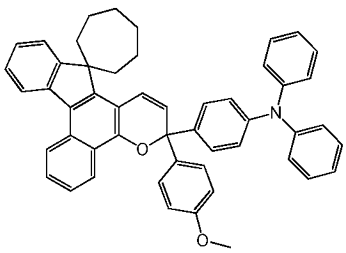
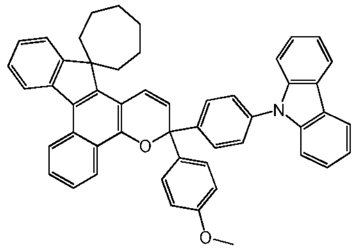
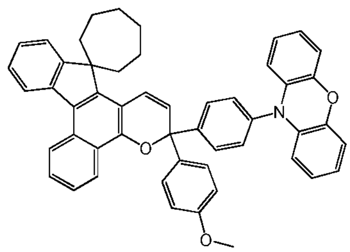
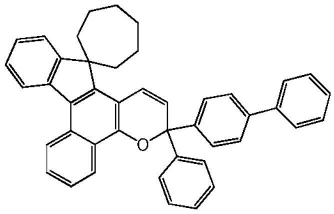
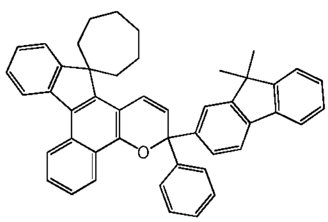
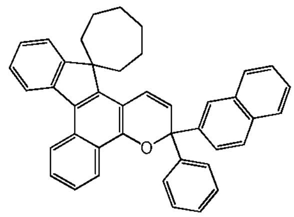

[Chem. 12]

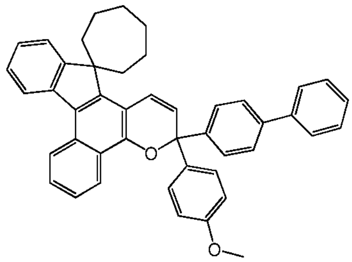
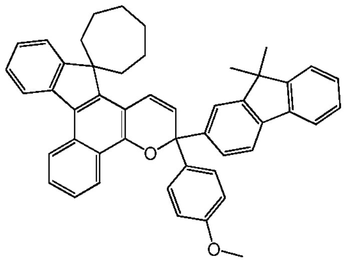
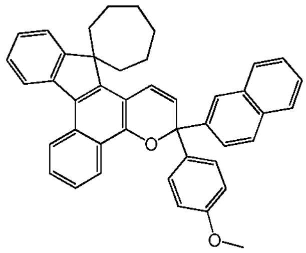
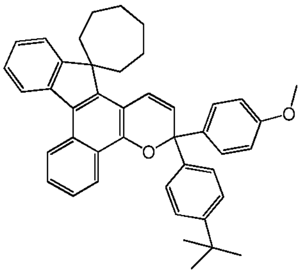
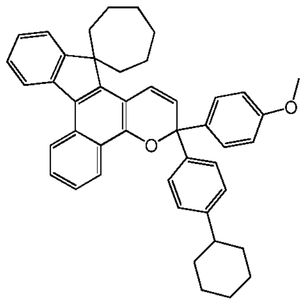
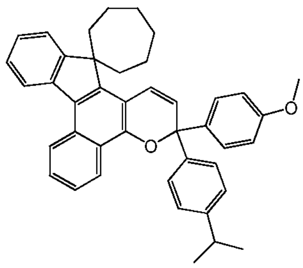
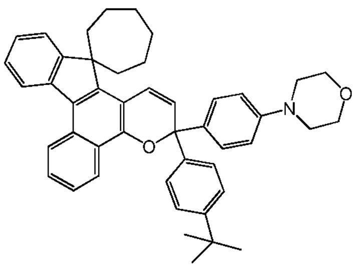
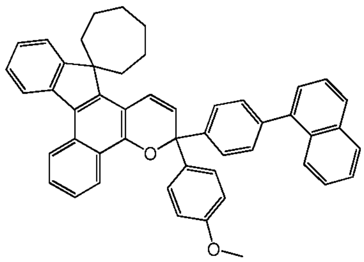
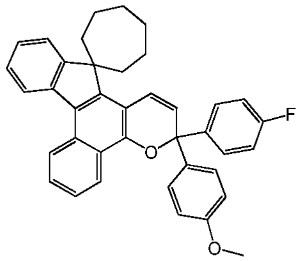
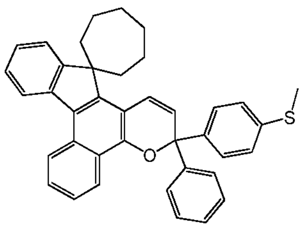
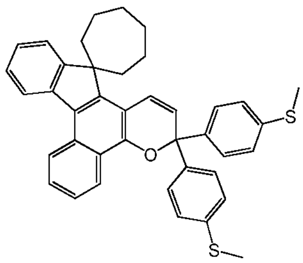
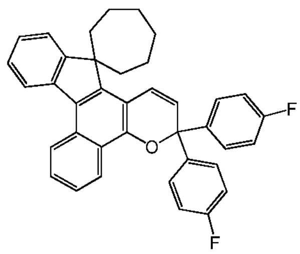
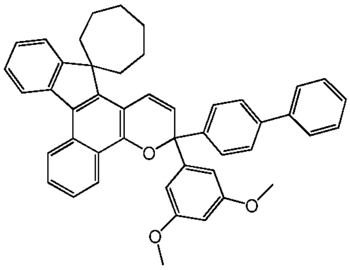
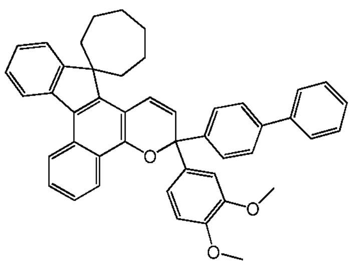

The photochromic compounds represented by General Formula 1 can be synthesized through a well-known method. For the synthesis method, for example, the following literature can be referred to. Japanese Patent No. 4884578, US 2006/0226402 A1, US 2006/0228557 A1, US 2008/0103301 A1, US 2011/0108781 A1, US 2011/0108781 A1, U.S. Patent Specification No. 7527754, U.S. Patent Specification No. 7556751, WO 2001/60811 A1, WO 2013/086248 A1, WO 1996/014596 A1, WO 2001/019813 A1, WO 1995/16215 A1, U.S. Patent Specification No. 5656206, and WO 2011/016582 A1

[Photochromic composition and photochromic article]

In addition, one aspect of the present invention relates to a photochromic composition containing one or more photochromic compounds represented by General Formula 1. In addition, one aspect of the present invention relates to a photochromic article containing one or more photochromic compounds represented by General Formula 1.

The above-described photochromic composition and the above-described photochromic article can contain only one of the photochromic compounds represented by General Formula 1 or two or more (for example, two or more and four or less) thereof. The above-described photochromic article and the above-described photochromic composition can contain, for example, about 0.1 to 15.0 mass% of the photochromic compounds represented by General Formula 1 based on the total amount of 100 mass% thereof. However, the present invention is not limited to the above-described range.

The above-described photochromic article can have at least a substrate. In one form, the photochromic compounds represented by General Formula 1 can be included in the substrate of the photochromic article. The above-described photochromic article can have a substrate and a photochromic layer, and the substrate and/or the photochromic layer can contain one or more photochromic compounds represented by General Formula 1. In the substrate and the photochromic layer, the photochromic compounds represented by General Formula 1 can be contained only in the substrate in one form, can be contained only in the photochromic layer in another form, and can be contained in the substrate and the photochromic layer in still another form. In addition, the substrate and the photochromic layer can contain, as photochromic compounds, only the photochromic compounds represented by General Formula 1 or one or more other photochromic compounds. Examples of other photochromic compounds include azobenzenes, spiropyrans, spirooxazines, naphthopyrans, indenonaphthopyrans, phenanthropyrans, hexaarylbisimidazoles, donor-acceptor Stenhouse adducts (DASA), salicylideneanilines, dihydropyrenes, anthracene dimers, fulgides, diarylethenes, phenoxynaphthacenequinones, and stilbenes.

<Substrate>

The above-described photochromic article can contain a substrate selected according to the type of photochromic article. Examples of substrates include spectacle lens substrates such as a plastic lens substrate and a glass lens substrate. The glass lens substrate can be, for example, a lens substrate made of inorganic glass. Examples of plastic lens substrates include: styrene resins such as a (meth)acrylic resin; allyl carbonate resins such as a polycarbonate resin, an allyl resin, and diethylene glycol bisallyl carbonate resin (CR-39); vinyl resins; polyester resins; polyether resins; urethane resins obtained by reacting an isocyanate compound with a hydroxy compound such as diethylene glycol; thiourethane resins obtained by reacting an isocyanate compound with a polythiol compound; and cured products obtained by curing a curable composition containing a (thio)epoxy compound having one or more disulfide bonds in the molecule (generally referred to as transparent resins). As lens substrates, unstained substrates (colorless lenses) may be used or stained substrate (stained lenses) may be used.
The refractive index of a lens substrate may be, for example, about 1.50 to 1.75. However, the refractive index of the lens substrate is not limited to the above-described range, and may be within, above, or below the above-described range. Here, the refractive index refers to a refractive index for light having a wavelength of 500 nm. In addition, the lens substrate may be a lens having index (so-called prescription lens) or a lens having no index (so-called non-prescription lens) .

For example, the above-described photochromic composition can be a polymerizable composition. In the present invention and the present specification, the term "polymerizable composition" is a composition containing one or more polymerizable compounds. A polymerizable composition containing at least one or more photochromic compounds represented by General Formula 1 and one or more polymerizable compounds can be molded through a well-known molding method to produce a cured product of such a polymerizable composition. Such a cured product can be included as a substrate in the above-described photochromic article and/or can be included as a photochromic layer. The curing treatment can be light emission and/or heat treatment. The polymerizable compound is a compound having a polymerizable group, and as a polymerization reaction of the polymerizable compound proceeds, the polymerizable composition can be cured to form a cured product. The polymerizable composition can further contain one or more additives (for example, a polymerization initiator).

Spectacle lenses may include various lenses such as a single focal lens, a multifocal lens, and a progressive power lens. The type of lens is determined by the surface shape of both sides of a lens substrate. In addition, the surface of a lens substrate may be a convex surface, a concave surface, or a flat surface. In general lens substrates and spectacle lenses, the object-side surface is a convex surface and the eyeball-side surface is a concave surface. However, the present invention is not limited thereto. A photochromic layer may be generally provided on the object-side surface of a lens substrate, or may be provided on the eyeball-side surface.

<Photochromic layer>

The photochromic layer can be a layer directly provided on the surface of a substrate or indirectly provided via one or more other layers. The photochromic layer can be, for example, a cured layer obtained by curing a polymerizable composition. A photochromic layer can be formed as a cured layer obtained by curing a polymerizable composition containing at least one or more photochromic compounds represented by General Formula 1 and one or more polymerizable compounds. For example, by directly applying such a polymerizable composition to the surface of a substrate or applying it to the surface of a layer provided on a substrate and curing the applied polymerizable composition, a photochromic layer can be formed as a cured layer containing one or more photochromic compounds represented by General Formula 1. As the coating method, well-known coating methods such as a spin coating method, a dip-coating method, a spray coating method, an ink jet method, a nozzle coating method, and a slit coating method can be employed. The curing treatment can be light emission and/or heat treatment. The polymerizable composition can further contain one or more additives (for example, a polymerization initiator) in addition to one or more polymerizable compounds. As a polymerization reaction of the polymerizable compound proceeds, the polymerizable composition can be cured to form a cured layer.

The thickness of the photochromic layer can be, for example, 5 um or more, 10 um or more, or 20 um or more, and can be, for example, 80 um or less, 70 um or less, or 50 um or less.

<Polymerizable compound>

In the present invention and the present specification, the polymerizable compound refers to a compound having one or more polymerizable groups in one molecule, and the term "polymerizable group" refers to a reactive group that can undergo a polymerization reaction. Examples of polymerizable groups include an acryloyl group, a methacryloyl group, a vinyl group, a vinyl ether group, an epoxy group, a thiol group, an oxetane group, a hydroxy group, a carboxy group, an amino group, and an isocyanate group.

Examples of polymerizable compounds that can be used to form the above-described substrate and the above-described photochromic layer include the following compounds.

(Episulfide compound)

The episulfide compound is a compound having two or more episulfide groups in one molecule. The episulfide groups are polymerizable groups that can undergo ring-opening polymerization. Specific examples of episulfide compounds include bis(1,2-epithioethyl) sulfide, bis(1,2-epithioethyl) disulfide, bis(2,3-epithiopropyl) sulfide, bis(2,3-epithiopropylthio)methane, bis(2,3-epithiopropyl) disulfide, bis(2,3-epithiopropyldithio)methane, bis(2,3-epithiopropyldithio)ethane, bis(6,7-epithio-3,4-dithiaheptyl) sulfide, bis(6,7-epithio-3,4-dithiaheptyl) disulfide, 1,4-dithiane-2,5-bis(2,3-epithiopropyldithiomethyl), 1,3-bis(2,3-epithiopropyldithiomethyl)benzene, 1,6-bis(2,3-epithiopropyldithiomethyl)-2-(2,3-epithiopropyldithioethylthio)-4-thiahexane, 1,2,3-tris(2,3-epithiopropyldithio)propane, 1,1,1,1-tetrakis(2,3-epithiopropyldithiomethyl)methane, 1,3-bis(2,3-epithiopropyldithio)-2-thiapropane, 1,4-bis(2,3-epithiopropyldithio)-2,3-dithiabutane, 1,1,1-tris(2,3-epithiopropyldithio)methane, 1,1,1-tris(2,3-epithiopropyldithiomethylthio)methane, 1,1,2,2-tetrakis(2,3-epithiopropyldithio)ethane, 1,1,2,2-tetrakis(2,3-epithiopropyldithiomethylthio)ethane, 1,1,3,3-tetrakis(2,3-epithiopropyldithio)propane, 1,1,3,3-tetrakis(2,3-epithiopropyldithiomethylthio)propane, 2-[1,1-bis(2,3-epithiopropyldithio)methyl]-1,3-dithietane, and 2-[1,1-bis(2,3-epithiopropyldithiomethylthio)methyl]-1,3-dithietane.

(Thietanyl compound)

The thietanyl compound is a thietane compound having two or more thietanyl groups in one molecule. The thietanyl group is a polymerizable group that can undergo ring-opening polymerization. Some thietanyl compounds have an episulfide group together with a plurality of thietanyl groups. Such compounds are listed as examples of the above-described episulfide compounds. Other thietanyl compounds include metal-containing thietane compounds having metal atoms in the molecule and non-metallic thietane compounds containing no metal.

Specific examples of non-metallic thietane compounds include bis(3-thietanyl) disulfide, bis(3-thietanyl) sulfide, bis(3-thietanyl) trisulfide, bis(3-thietanyl) tetrasulfide, 1,4-bis(3-thietanyl)-1,3,4-trithiabutane, 1,5-bis(3-thietanyl)-1,2,4,5-tetrathiapentane, 1,6-bis(3-thietanyl)-1,3,4,6-tetrathiahexane, 1,6-bis(3-thietanyl)-1,3,5,6-tetrathiahexane, 1,7-bis(3-thietanyl)-1,2,4,5,7-pentathiaheptane, 1,7-bis(3-thietanylthio)-1,2,4,6,7-pentathiaheptane, 1,1-bis(3-thietanylthio)methane, 1,2-bis(3-thietanylthio)ethane, 1,2,3-tris(3-thietanylthio)propane, 1,8-bis(3-thietanylthio)-4-(3-thietanylthiomethyl)-3,6-dithiaoctane, 1,11-bis(3-thietanylthio)-4,8-bis(3-thietanylthiomethyl)-3,6,9-trithiaundecane, 1,11-bis(3-thietanylthio)-4,7-bis(3-thietanylthiomethyl)-3,6,9-trithiaundecane, 1,11-bis(3-thietanylthio)-5,7-bis(3-thietanylthiomethyl)-3,6,9-trithiaundecane, 2,5-bis(3-thietanylthiomethyl)-1,4-dithiane, 2,5-bis[[2-(3-thietanylthio)ethyl]thiomethyl]-1,4-dithiane, 2,5-bis(3-thietanylthiomethyl)-2,5-dimethyl-1,4-dithiane, bis-thietanyl sulfide, bis(thietanylthio) methane, 3-[<(thietanylthio)methylthio>methylthio]thietane, bis-thietanyl disulfide, bis-thietanyl trisulfide, bis-thietanyl tetrasulfide, bis-thietanyl pentasulfide, 1,4-bis(3-thietanyldithio)-2,3-dithibutane, 1,1,1-tris(3-thietanyldithio)methane, 1,1,1-tris(3-thietanyldithiomethylthio)methane, 1,1,2,2-tetrakis(3-thietanyldithio)ethane, and 1,1,2,2-tetrakis(3-thietanyldithiomethylthio) ethane.

Examples of metal-containing thietane compounds include, as metal atoms in the molecule, those containing the Group 14 atoms such as an Sn atom, an Si atom, a Ge atom, and a Pb atom, the Group 4 elements such as a Zr atom and a Ti atom, the Group 13 atoms such as an Al atom, and the Group 12 atoms such as a Zn atom. Specific examples thereof include alkylthio(thietanylthio)tin,

bis(alkylthio)bis(thietanylthio)tin,
alkylthio(alkylthio)bis(thietanylthio)tin,
bis(thietanylthio)cyclic dithiotin compounds, and
alkyl(thietanylthio)tin compounds.

Specific examples of alkylthio(thietanylthio)tin include methylthiotris(thietanylthio)tin,

ethylthiotris(thietanylthio)tin,
propylthiotris(thietanylthio)tin, and
isopropylthiotris(thietanylthio)tin.

Specific examples of bis(alkylthio)bis(thietanylthio)tin include bis(methylthio)bis(thietanylthio)tin,

bis(ethylthio)bis(thietanylthio)tin,
bis(propylthio)bis(thietanylthio)tin, and
bis(isopropylthio)bis(thietanylthio)tin.

Specific examples of

alkylthio(alkylthio)bis(thietanylthio)tin include
ethylthio(methylthio)bis(thietanylthio)tin,
methylthio(propylthio)bis(thietanylthio)tin,
isopropylthio(methylthio)bis(thietanylthio)tin,
ethylthio(propylthio)bis(thietanylthio)tin,
ethylthio(isopropylthio)bis(thietanylthio)tin, and
isopropylthio(propylthio)bis(thietanylthio)tin.

Specific examples of bis(thietanylthio)cyclic dithiotin compounds include bis(thietanylthio)dithiastannetane, bis(thietanylthio)dithiastannolane,

bis(thietanylthio)dithiastanninane, and
bis(thietanylthio)trithiastannocane.

Specific examples of alkyl(thietanylthio)tin compounds include methyltris(thietanylthio)tin, dimethylbis(thietanylthio)tin, butyltris(thietanylthio)tin, tetrakis(thietanylthio)tin, tetrakis(thietanylthio)germanium, and tris(thietanylthio)bismuth.

(Polyamine compound)

The polyamine compound is a compound having two or more $NH_2$ groups in one molecule, and can form a urea bond according to a reaction with a polyisocyanate and can form a thiourea bond according to a reaction with a polyisothiocyanate. Specific examples of polyamine compounds include ethylenediamine, hexamethylenediamine, isophoronediamine, nonamethylenediamine, undecamethylenediamine, dodecamethylenediamine, metaxylenediamine, 1,3-propanediamine, putrescine, 2-(2-aminoethylamino)ethanol, diethylenetriamine, p-phenylenediamine, m-phenylenediamine, melamine, and 1,3,5-benzenetriamine.

(Epoxy compound)

The epoxy compound is a compound having an epoxy group in the molecule. The epoxy group is a polymerizable group that can undergo ring-opening polymerization. Epoxy compounds are generally classified into aliphatic epoxy compounds, alicyclic epoxy compounds, and aromatic epoxy compounds.

Specific examples of aliphatic epoxy compounds include ethylene oxide, 2-ethyloxirane, butyl glycidyl ether, phenyl glycidyl ether, 2,2'-methylenebisoxirane, 1,6-hexanediol diglycidyl ether, ethylene glycol diglycidyl ether, diethylene glycol diglycidyl ether, triethylene glycol diglycidyl ether, tetraethylene glycol diglycidyl ether, nonaethylene glycol diglycidyl ether, propylene glycol diglycidyl ether, dipropylene glycol diglycidyl ether, tripropylene glycol diglycidyl ether, tetrapropylene glycol diglycidyl ether, nonapropylene glycol diglycidyl ether, neopentyl glycol diglycidyl ether, trimethylolpropane triglycidyl ether, glycerol triglycidyl ether, diglycerol tetraglycidyl ether, pentaerythritol tetraglycidyl ether, and tris(2-hydroxyethyl)isocyanurate triglycidyl ether.

Specific examples of alicyclic epoxy compounds include isophoronediol diglycidyl ether and bis-2,2-hydroxycyclohexylpropane diglycidyl ether.

Specific examples of aromatic epoxy compounds include resorcinol diglycidyl ether, bisphenol A diglycidyl ether, bisphenol F diglycidyl ether, bisphenol S diglycidyl ether, diglycidyl orthophthalate, phenol novolac polyglycidyl ether, and cresol novolac polyglycidyl ether.

In addition to the above-described examples, an epoxy compound having a sulfur atom in the molecule together with an epoxy group can be used. Such epoxy compounds containing sulfur atoms include linear aliphatic compounds and cycloaliphatic compounds.

Specific examples of linear aliphatic epoxy compounds containing sulfur atoms include bis(2,3-epoxypropyl) sulfide, bis(2,3-epoxypropyl) disulfide, bis(2,3-epoxypropylthio)methane, 1,2-bis(2,3-epoxypropylthio)ethane, 1,2-bis(2,3-epoxypropylthio)propane, 1,3-bis(2,3-epoxypropylthio)propane, 1,3-bis(2,3-epoxypropylthio)-2-methylpropane, 1,4-bis(2,3-epoxypropylthio)butane, 1,4-bis(2,3-epoxypropylthio)-2-methylbutane, 1,3-bis(2,3-epoxypropylthio)butane, 1,5-bis(2,3-epoxypropylthio)pentane, 1,5-bis(2,3-epoxypropylthio)-2-methylpentane, 1,5-bis(2,3-epoxypropylthio)-3-thiapentane, 1,6-bis(2,3-epoxypropylthio)hexane, 1,6-bis(2,3-epoxypropylthio)-2-methylhexane, 3,8-bis(2,3-epoxypropylthio)-3,6-dithiaoctane, 1,2,3-tris(2,3-epoxypropylthio)propane, 2,2-bis(2,3-epoxypropylthio)-1,3-bis(2,3-epoxypropylthiomethyl)propane, and 2,2-bis(2,3-epoxypropylthiomethyl)-1-(2,3-epoxypropylthio)butane.

Specific examples of cycloaliphatic epoxy compounds containing sulfur atoms include 1,3-bis(2,3-epoxypropylthio)cyclohexane, 1,4-bis(2,3-epoxypropylthio)cyclohexane, 1,3-bis(2,3-epoxypropylthiomethyl)cyclohexane, 1,4-bis(2,3-epoxypropylthiomethyl)cyclohexane, 2,5-bis(2,3-epoxypropylthiomethyl)-1,4-dithiane, 2,5-bis[<2-(2,3-epoxypropylthio)ethyl>thiomethyl]-1,4-dithiane, and 2,5-bis(2,3-epoxypropylthiomethyl)-2,5-dimethyl-1,4-dithiane.

(Compound having radically polymerizable group)

The radically polymerizable group is a polymerizable group that can undergo radical polymerization. Examples of radically polymerizable groups include an acryloyl group, a methacryloyl group, an allyl group, and a vinyl group.

In the following, a compound having a polymerizable group selected from the group consisting of an acryloyl group and a methacryloyl group will be referred to as a "(meth)acrylate compound." Specific examples of (meth)acrylate compounds include ethylene glycol di(meth)acrylate, diethylene glycol di(meth)acrylate, triethylene glycol di(meth)acrylate, tetraethylene glycol di(meth)acrylate, polyethylene glycol di(meth)acrylate, propylene glycol di(meth)acrylate, dipropylene glycol di(meth)acrylate, tripropylene glycol di(meth)acrylate, polypropylene glycol di(meth)acrylate, neopentyl glycol di(meth)acrylate, ethylene glycol bisglycidyl(meth)acrylate), bisphenol A di(meth)acrylate, 2,2-bis(4-(meth)acryloxyethoxyphenyl)propane, 2,2-bis(4-(meth)acryloxydiethoxyphenyl)propane, 2,2-bis(4-(meth)acryloyloxyethoxyphenyl)propane, 2,2-bis(3,5-dibromo-4-(meth)acryloyloxyethoxyphenyl)propane, 2,2-bis(4-(meth)acryloyloxydipropoxyphenyl)propane, bisphenol F di(meth)acrylate, 1,1-bis(4-(meth)acryloxyethoxyphenyl)methane, 1,1-bis(4-(meth)acryloxydiethoxyphenyl)methane, dimethyloltricyclodecane di(meth)acrylate, trimethylolpropane tri(meth)acrylate, ditrimethylolpropane tetra(meth)acrylate, glycerol di(meth)acrylate, pentaerythritol tri(meth)acrylate, pentaerythritol tetra(meth)acrylate, methylthio(meth)acrylate, phenylthio(meth)acrylate, benzylthio(meth)acrylate, xylylene dithiol di(meth)acrylate, mercaptoethyl sulfide di(meth)acrylate, and difunctional urethane (meth)acrylate.

Specific examples of compounds having an allyl group (allyl compounds) include allyl glycidyl ether, diallyl phthalate, diallyl terephthalate, diallyl isophthalate, diallyl carbonate, diethylene glycol bisallyl carbonate, methoxy polyethylene glycol allyl ether, polyethylene glycol allyl ether, methoxy polyethylene glycol-polypropylene glycol allyl ether, butoxy polyethylene glycol-polypropylene glycol allyl ether, methacryloyloxy polyethylene glycol-polypropylene glycol allyl ether, phenoxy polyethylene glycol allyl ether, and methacryloyloxy polyethylene glycol allyl ether.

Examples of compounds having a vinyl group (vinyl compounds) include $\alpha$-methylstyrene, $\alpha$-methylstyrene dimer, styrene, chlorostyrene, methylstyrene, bromostyrene, dibromostyrene, divinylbenzene, and 3,9-divinylspirobi(m-dioxane).

The above-described photochromic article can include one or more layers well known as functional layers of photochromic articles such as a protective layer for improving the durability of a photochromic article, an antireflection layer, a water-repellent or hydrophilic antifouling layer, an antifogging layer, and a primer layer for improving adhesion between layers at any position.

The above-described photochromic article can be an optical article. One form of the optical article is a spectacle lens. Such a spectacle lens can also be called a photochromic lens or a photochromic spectacle lens. In addition, as one form of the optical article, a goggle lens, a visor (cap) part of a sun visor, a shield member of a helmet, and the like can be exemplified. An optical article having an anti-glare function can be obtained by applying the above-described photochromic composition which is a polymerizable composition to a substrate for such an optical article and curing the applied composition to form a photochromic layer.

[Spectacles]

One aspect of the present invention relates to spectacles including spectacle lenses that are one form of the above-described photochromic article. The details of the spectacle lenses included in the spectacles are as described above. By equipping such spectacle lenses, for example, the above-described spectacles can exhibit an anti-glare effect like sunglasses when the photochromic compound is colored by exposure to sunlight outdoors, and the photochromic compound can fade upon returning indoors, and thus the transmittance can be recovered. For the above-described spectacles, a well-known technique can be applied to the configuration of the frame and the like.

[Examples]

Hereinafter, the present invention will be described in more detail with reference to examples. However, the present invention is not limited to embodiment shown in the examples.

In the following, the molecular structure was identified using a nuclear magnetic resonance (NMR) apparatus. Proton NMR of ECS-400 (manufactured by JEOL Ltd.) was used as NMR. As a measurement solvent, deuterated chloroform was mainly used, and deuterated dimethyl sulfoxide, deuterated acetone, deuterated acetonitrile, deuterated benzene deuterated methanol, deuterated pyridine, or the like was appropriately used.

The purity was analyzed using high-performance liquid chromatography (HPLC). LC-2040C manufactured by Shimadzu Corporation was used as HPLC. YMC-Triart C18 was used as a column, and the measurement temperature was set to 40°C. A mixed solvent of acetonitrile and water containing 0.1% or trifluoroacetic acid was used as a mobile phase, and the flow rate was set to 0.4 mL/min.

For mass spectrometry, an apparatus equipped with SQD2 was used as a mass spectrometry unit in ACQUITY UPLC H-Class system (UPLC) manufactured by Nihon Waters K.K. ACQUITY UPLC BEH C18 was used as a column, and the measurement temperature was set to 40°C. A mixed solvent of acetonitrile and water containing formic acid was used as a mobile phase and allowed to flow at a flow rate of 0.61 mL/min with a concentration gradient. An electrospray ionization (ESI) method was used for ionization.

CHN (carbon, hydrogen, and nitrogen) element analysis was conducted through a combustion method.

[Example 1]

From the reaction products shown in Table 1, the following exemplary compound 1 was obtained through the following method.

In an argon atmosphere, p-toluenesulfonic acid monohydrate (0.15 g, 0.80 mmol) was added to a toluene solution (36 mL) containing a reaction product 1 (1.2 g, 4 mmol) and a reaction product 2 (1.9 g, 8 mmol) shown in Table 1, and the mixture was stirred at room temperature overnight. An aqueous sodium hydroxide solution (1.0 M, 37 mL) was added thereto, and the mixture was stirred for about 20 minutes. Impurities were removed by filtration, extraction with toluene (30 mL×2) was performed, and the combined organic layer was then washed with water (20 mL×2) and concentrated. The obtained residue was purified through column chromatography ($SiO_2$: 200 g, heptane/chloroform (volume basis)=70/30 to 60/40) (1.2 g, brown solid). The obtained solid was suspended in heptane/ethyl acetate (2/1 (volume basis), 90 mL), subjected to ultrasound treatment for about 30 minutes, and filtered and dried to obtain an exemplary compound 1 as a light yellow-green solid (0.9 g) which is a final product.

The obtained product was analyzed by the following method.

The structure was identified by a nuclear magnetic resonance (NMR) apparatus.

The purity was analyzed by HPLC and was a value shown in Table 1 in terms of area ratio.

As a result of mass spectrometry, the actual measurement value (M+, relative intensity of 100) was shown in Table 1 for the calculation value of the precise mass shown in Table 1.

As a result of CHN elemental analysis by a combustion method, the actual measurement value was a value

shown in Table 1 for the calculation value shown in Table 1.

It was confirmed based on the above analysis results that the exemplary compound 1 as a target compound was produced comprehensively.

[Examples 2 to 5 and Comparative Example 1]

Exemplary compounds 2 to 5 and a comparative compound 1 were obtained through the same operation as described above except that reaction products shown in Table 1 were used as the reaction product 1 and the reaction product 2 used to synthesize compounds.

The obtained products were analyzed by the method described above. The analysis results are shown in Table 1.

[Chem. 13]

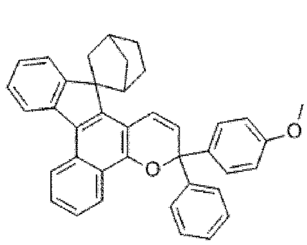

Exemplary compound 1

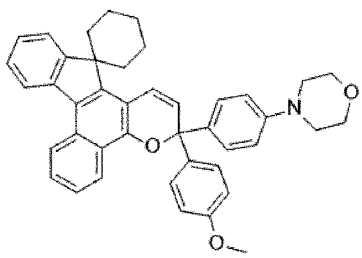

Exemplary compound 2

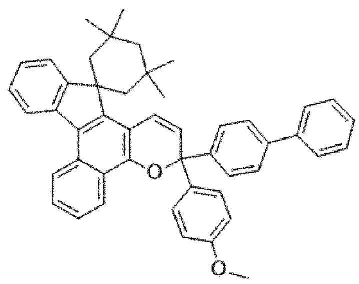

Exemplary compound 3

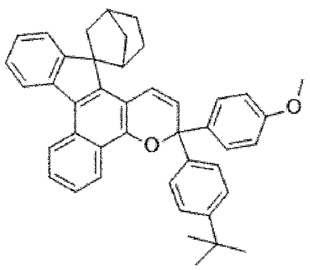

Exemplary compound 4

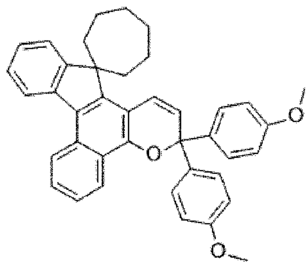

Exemplary compound 5

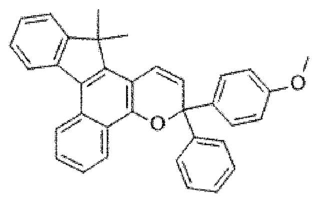

Comparative compound 1

[Evaluation method]

<Measurement of solution spectrum and evaluation of fading rate>

For Examples 1 to 5 and Comparative Example 1, each compound was dissolved in chloroform containing no stabilizer to prepare a chloroform solution containing the compound.

A 1 cm square quartz spectroscopic cell containing the prepared solution was covered, and ultraviolet rays were emitted as an ultraviolet light source for 15 seconds using UV-LED (a combination of LIGHTNINGCURE LC-L1V5 and L14310-120, an output of 70%) manufactured by Hamamatsu Photonics K.K. The solution was stirred with a small stirrer during ultraviolet emission. Within 10 seconds after ultraviolet emission was completed, the absorbance was measured using a UV-visible spectrophotometer (UV-1900i manufactured by Shimadzu Corporation, measurement wavelength of 700 to 400 nm, wavelength intervals of 2 nm, survey mode). The absorbance was measured at room temperature (23°C to 28°C). The concentration of the solution was adjusted so that the absorbance at the first absorption wavelength (the peak of the absorption intensity observed at the longest wavelength) was 0.95 to 1.05. Furthermore, the absorbance was measured every 10 seconds, and the attenuation of the absorbance was measured. Normalization was performed so that the peak of the first absorption wavelength in the first absorbance measurement became 1, the attenuation of the absorbance was then measured, data of fading for initial 100 seconds (11 absorbance measurements) from the change in

absorbance over time was analyzed with a first-order reaction model, and thus the reaction rate constant was obtained. If $[A_0]$ is an initial concentration of a colored body, that is, a normalized absorbance value of 1, [A] is a concentration of the colored body after a certain period of time, that is, a normalized absorbance value, t is time (seconds), and k is a rate constant, the first-order reaction can be expressed as in the following equation. This equation is called an integral form velocity equation.

[Math. 1]

$$\ln\frac{[A]}{[A_0]} = -kt$$

Table 1 shows reaction rate constants obtained for Examples 1 to 5 and Comparative Example 1. It was possible to confirm from the results shown in Table 1 that respective compounds of Examples 1 to 5 had a higher fading rate than the comparative compound 1 of Comparative Example 1.

In addition, in the above-described measurement, it was confirmed that the compounds of all examples exhibited photochromic properties of structural transition to a molecular structure with strong absorption in a visible range upon emission of ultraviolet rays.

[Table 1]

| | Reaction product 1 | Reaction product 2 | HPLC purity (%) | Calculated precise mass | Actual measurement value of mass spectrometry | Calculation value of CHN composition (%) | Actual measurement value of CHN elemental analysis (%) | Reaction rate constant ($10^{-3}$ $sec^{-1}$) |
|---|---|---|---|---|---|---|---|---|
| Example 1 | 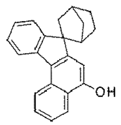 | 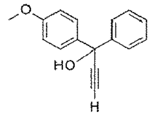 | 96% | 532. 240 | 533.1 | C: 87.9% H: 6.1% | C: 88.0% H: 5.2% | 10.2 |
| Example 2 | 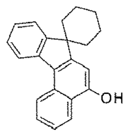 | 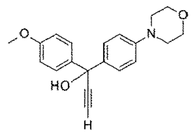 | 95% | 605. 293 | 605.4 | C: 83.3% H: 6.5% N: 2.3% | C: 82.3% H: 6.6% N: 2.1% | 11.2 |
| Example 3 | 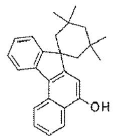 | 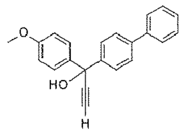 | 98% | 652. 334 | 652.5 | C: 88.3% H: 6.8% | C: 87.9% H: 6.3% | 8.8 |
| Example 4 | 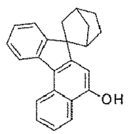 | 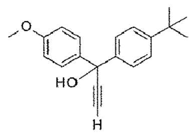 | 96% | 588. 303 | 588.5 | C: 87.7% H: 6.8% | C: 87.4% H: 6.6% | 10.5 |
| Example 5 | 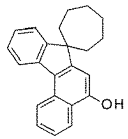 | 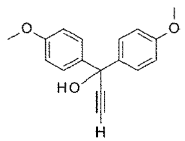 | 98% | 564. 266 | 564. 5 | C: 85.1% H: 6.4% | C: 86.0% H: 6.7% | 9.2 |
| Comparative Example 1 | 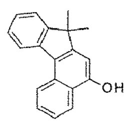 | 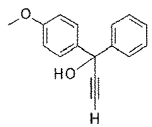 | 98% | 480. 209 | 480. 3 | C: 87.5% H: 5.9% | C: 86.8% H: 6.3% | 2.8 |

Finally, the above-described aspects are summarized.

According to one aspect, a photochromic compound represented by General Formula 1 is provided.

In one form, in General Formula 1, $R^1$ to $R^4$ can each independently represent a hydrogen atom or a substituent other than an electron-withdrawing group.

In one form, in General Formula 1, $R^1$ to $R^4$ can all represent a hydrogen atom.

According to one aspect, a photochromic composition containing the above-described photochromic compound is provided.

In one form, the above-described photochromic composition can further contain a polymerizable compound.

According to one aspect, a photochromic article containing a cured product obtained by curing the above-described photochromic composition is provided.

In one form, the above-described photochromic article can have a substrate and a photochromic layer that is the above-described cured product.

In one form, the above-described photochromic article can be a spectacle lens.

In one form, the above-described photochromic article can be a goggle lens.

In one form, the above-described photochromic article can be a visor part of a sun visor.

In one form, the above-described photochromic article can be a shield member of a helmet.

According to one aspect, spectacles having the above-described spectacle lens are provided.

Two or more of various aspects and various forms described in the present specification can be combined in arbitrary combinations.

The embodiment disclosed herein is only an example in all respects and should not be considered as restrictive. The scope of the present invention is indicated by the claims, not by the above description, and is intended to include the meaning equivalent to the claims and all modifications within the scope of the claims.

[Industrial Applicability]

One aspect of the present invention is useful in the technical fields of spectacles, goggles, sun visors, helmets, and the like.

## Claims

1. A photochromic compound represented by General Formula 1 below,

[Chem. 1]

General Formula 1

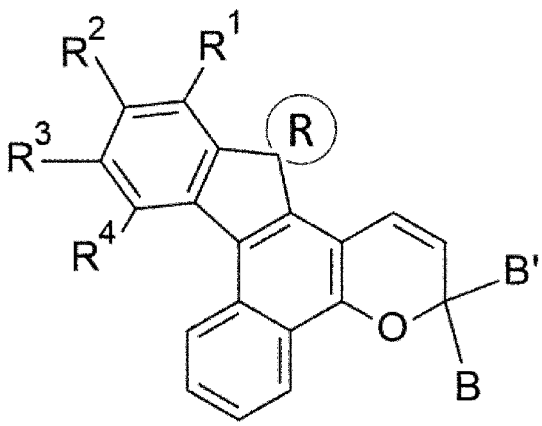

(in General Formula 1,

R represents a substituted or unsubstituted ring structure which is spirofused with an indeno-fused naphthopyran and has 3 or more and 20 or less carbon atoms (including the carbon atom at the 13-position of the indeno-fused naphthopyran), and
$R^1$ to $R^4$, B, and B' each independently represent a hydrogen atom or a substituent.)

**2.** The photochromic compound according to claim 1, wherein $R^1$ to $R^4$ each independently represent a hydrogen atom or a substituent other than an electron-withdrawing group.

**3.** The photochromic compound according to claim 1 or 2, wherein, $R^1$ to $R^4$ all represent a hydrogen atom.

**4.** A photochromic composition comprising: the photochromic compound according to any one of claims 1 to 3.

**5.** The photochromic composition according to claim 4, further comprising: a polymerizable compound.

**6.** A photochromic article comprising: a cured product obtained by curing the photochromic composition according to claim 5.

**7.** The photochromic article according to claim 6, further comprising:

a substrate; and
a photochromic layer which is the cured product.

**8.** The photochromic article according to claim 6 or 7, which is a spectacle lens.

**9.** The photochromic article according to claim 6 or 7, which is a goggle lens.

**10.** The photochromic article according to claim 6 or 7, which is a visor part of a sun visor.

**11.** The photochromic article according to claim 6 or 7, which is a shield member of a helmet.

**12.** Spectacles comprising: the spectacle lens according to claim 8.

**INTERNATIONAL SEARCH REPORT**

International application No. **PCT/JP2023/003028**

**A. CLASSIFICATION OF SUBJECT MATTER**

***C07D 413/10***(2006.01)i; ***C09K 9/02***(2006.01)i; ***G02C 7/10***(2006.01)i; ***C07D 311/04***(2006.01)i; ***C09B 57/00***(2006.01)i
FI: C09K9/02 B; C09B57/00 Z; C07D413/10; C07D311/04; G02C7/10

According to International Patent Classification (IPC) or to both national classification and IPC

**B. FIELDS SEARCHED**

Minimum documentation searched (classification system followed by classification symbols)

C07D413/10; C09K9/02; G02C7/10; C07D311/04; C09B57/00

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| X | WO 2008/105306 A1 (TOKUYAMA CORPORATION) 04 September 2008 (2008-09-04) paragraphs [0119]-[0132], [0162]-[0177], tables 5-7, examples | 1-8, 12 |
| Y | paragraphs [0119]-[0132], [0162]-[0177], tables 5-7, examples | 9-11 |
| X | WO 2009/075388 A1 (TOKUYAMA CORPORATION) 18 June 2009 (2009-06-18) pp. 40-62, tables 1-11, examples | 1-8, 12 |
| Y | pp. 40-62, tables 1-11, examples | 9-11 |
| X | JP 2021-148814 A (TOKUYAMA CORPORATION) 27 September 2021 (2021-09-27) paragraphs [0250]-[0285], tables 1-4, examples | 1-8, 12 |
| Y | paragraphs [0250]-[0285], tables 1-4, examples | 9-11 |
| X | JP 2005-289807 A (TOKUYAMA CORPORATION) 20 October 2005 (2005-10-20) paragraphs [0120]-[0146], table 6, examples 1, 4, 6-11 | 1-8, 12 |
| Y | paragraphs [0120]-[0146], table 6, examples 1, 4, 6-11 | 9-11 |
| Y | WO 2020/203864 A1 (HOYA LENS THAILAND LTD) 08 October 2020 (2020-10-08) claims 5-15 | 9-11 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **24 March 2023** | **04 April 2023** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

**INTERNATIONAL SEARCH REPORT**

International application No.
**PCT/JP2023/003028**

**C. DOCUMENTS CONSIDERED TO BE RELEVANT**

| Category* | Citation of document, with indication, where appropriate, of the relevant passages | Relevant to claim No. |
|---|---|---|
| A | WO 2011/034202 A1 (TOKUYAMA CORPORATION) 24 March 2011 (2011-03-24) entire text, all drawings | 1-12 |

Form PCT/ISA/210 (second sheet) (January 2015)

**INTERNATIONAL SEARCH REPORT**
**Information on patent family members**

International application No.
**PCT/JP2023/003028**

| Patent document cited in search report | Publication date (day/month/year) | Patent family member(s) | Publication date (day/month/year) |
|---|---|---|---|
| WO 2008/105306 A1 | 04 September 2008 | US 2010/0110521 A1 paragraphs [0398]-[0411], [0491]-[0542], tables 5-7, examples | |
| WO 2009/075388 A1 | 18 June 2009 | US 2010/0230650 A1 paragraphs [0113]-[0117], tables 1-11, examples | |
| JP 2021-148814 A | 27 September 2021 | (Family: none) | |
| JP 2005-289807 A | 20 October 2005 | (Family: none) | |
| WO 2020/203864 A1 | 08 October 2020 | US 2022/0186064 A1 claims 5-15 | |
| WO 2011/034202 A1 | 24 March 2011 | US 2012/0170098 A1 entire text, all drawings | |

Form PCT/ISA/210 (patent family annex) (January 2015)

## REFERENCES CITED IN THE DESCRIPTION

*This list of references cited by the applicant is for the reader’s convenience only. It does not form part of the European patent document. Even though great care has been taken in compiling the references, errors or omissions cannot be excluded and the EPO disclaims all liability in this regard.*

### Patent documents cited in the description


- WO 200160811 A1
- JP 4884578 B
- US 20060226402 A1
- US 20060228557 A1
- US 20080103301 A1
- US 20110108781 A1
- US 7527754 B
- US 7556751 B
- WO 2013086248 A1
- WO 1996014596 A1
- WO 2001019813 A1
- WO 199516215 A1
- US 5656206 A
- WO 2011016582 A1


### Non-patent literature cited in the description


- Daigakuin Yuki Kagaku (Jou) (’’Graduate School Organic Chemistry (Part 1). 1988